# EUROPEAN PATENT SPECIFICATION

(11) **EP 0 960 567 B1**
(45) Date of publication and mention of the grant of the patent: **05.10.2005**
(21) Application number: 99202586.6
(22) Date of filing: 31.05.1996
(51) Int. Cl.: A23D 9/00, A23D 7/00, A23L 1/24

(54) **Fat based food products**
Fett enthaltende Nahrungsmittelprodukte
Produits alimentaires à base de graisses

(30) Priority: 01.06.1995 EP 95201444; 25.07.1995 EP 95202042
(43) Date of publication of application: 01.12.1999
(62) Divisional of application: 96917474.7
(73) Proprietor: UNILEVER N.V., 3000 DK Rotterdam (NL); UNILEVER PLC, London EC4P 4BQ (GB)
(72) Inventor: Lievense, Lourus Cornelis, 1370-000 Valinhos/SP (BR)
(74) Representative: Van Velzen, Maaike Mathilde

(56) References cited:
- EP-A- 0 619 952
- WO-A-91/17985
- WO-A-92/19640
- GB-A- 1 413 102
- DATABASE WPI Week 8305 Derwent Publications Ltd., London, GB; AN 83-10783K XP002012417 & JP 57 206336 A (AJINOMOTO KK), 17 December 1982 (1982-12-17)
- DATABASE WPI Week 9502 Derwent Publications Ltd., London, GB; AN 95-011745 XP002012416 & JP 06 298645 A (EISAI CO LTD), 25 October 1994 (1994-10-25)
- DATABASE WPI Week 8618 Derwent Publications Ltd., London, GB; AN 86-117166 XP002012415 & JP 61 058536 A (NIPPON OILS & FATS KK), 25 March 1986 (1986-03-25)
- DATABASE WPI Week 8623 Derwent Publications Ltd., London, GB; AN 86-146234 XP002012419 & JP 61 015647 A (ASAHI DENKA KOGYO), 23 January 1986 (1986-01-23)
- PATENT ABSTRACTS OF JAPAN vol. 11, no. 379 (C-463), 10 December 1987 (1987-12-10) & JP 62 148424 A (RIKEN VITAMIN CO LTD), 2 July 1987 (1987-07-02)
- C. RUKMINI ET AL.: "Nutritional and biochemical aspects of the hypolipidemic action of rice bran oil: a review" JOURNAL OF THE AMERICAN COLLEGE OF NUTRITION, vol. 10, no. 6, 1991, pages 593-601, XP000612280 US
- DATABASE WPI Week 8640 Derwent Publications Ltd., London, GB; AN 86-262875 XP002012418 & JP 61 192264 A (TSUKINO SHOKUHIN KO), 26 August 1986 (1986-08-26)

## Description

The present invention concerns a fat based food product comprising natural fat components which have a blood cholesterol lowering effect in amounts sufficient to obtain a blood cholesterol lowering effect if the food product is used according to the common needs of the consumer.

Such fat based food products are available on the market for quite some time, and are to a large extend based on the use of fat which comprises a particular amount of triacylglycerides rich in poly-unsaturated fatty acid (pufa) chains. In particular, the use of butter-like spreads comprising a significant amount of these triacylglycerides having poly- unsaturated fatty acid chains (hereinafter called pufa rich triglycerides) is well known in the western world to reduce high blood cholesterol levels.

The variation in the mean plasma total cholesterol concentration among populations is highly correlated with the variation in the extent of atherosclerosis and in the incidence of Coronary Heart Disease (CHD), which is one of the major causes of death in the Western society. Populations with a low cholesterol level (less than 180 mg/dl (4.7 mmol/L) are found to be less sensitive to atherosclerosis and coronary heart disease, whereas those with mean cholesterol levels above 220 mg/dl (5.7 mmol/L; hypercholesterolaemia) have increased rates of death due to CHD. Thus, a clear need for a method by which the cholesterol level can be lowered exists.

With the common products which comprise triglycerides rich in pufa, a particular amount of fat should be eaten on a daily bases to ensure a high enough intake of these triglycerides. A contribution of a minimal intake of pufa glycerides of 3 to 4 percent of the metabolizable energy is recommended.
However, if fat based food products are used in which part of the fat is dispensed with, for example by the use of thickeners and/or fat replacers, products with a low fat level such as low fat spreads comprising less than 40% fat would have a less blood cholesterol lowering effect than a product comprising 80% fat, given that a similar amount of pufa rich triglycerides is present in the fat. This is even more the case for the products which were even further reduced in their fat content, such as low fat spreads comprising less than 20% fat.

For this type of products, a need for further improvement of the cholesterol lowering effect in fat based food products exists. Also for fat based food products which up till now did not comprise pufa rich triglycerides, the presence of components which have a cholesterol lowering effect is desired.

On the other hand, also the daily consumption of full fat food products per se is decreasing, i.e. not solely by replacement of these products by low fat food products but also by a decreasing consumption of fat based food products as such. This is mainly due to the increasing awareness of the need to reduce the fat intake in general, wrongly based on the scientific consensus towards the necessity to reduce the consumption of triacylglycerides with saturated fatty acids in particular. Because of the reasons mentioned above, for cholesterol reducing PUFA-triglycerides containing fat based products a reasonable consumption is still preferred for the majority of the population in the majority of the effluent countries. Nevertheless, to compensate for the reduction in pufa through reduction of the consumption of triglycerides rich in pufa, also for those consumers that use high fat food products (e.g. 40-80% yellow fat spreads), which contain more than 30% pufa rich triglycerides on fat, in a decreasing extend, the presence of components that have an additional cholesterol lowering effect is highly preferred.

Also for those consumers for which a significant reduction in plasma cholesterol is desired, i.e. hypercholesterolaemia patients, the consumption of a high or low fat food product that contains more than 30% and preferably more than 45% pufa-triglycerides on fat and also natural components that have an additional cholesterol lowering effect would be most desirable.

As consumers these days have a clear preference for food products which are obtained from natural sources our invention deals with food products which contribute to the health of consumers by the use of ingredients obtained from natural vegetable, oil related sources and to include such particular natural ingredients in their context, i.e. in food products comprising ingredients similar to the source of the particular ingredients. In the context of vegetable oil and fat based products, we have now found that some particular natural non-triglyceride minor oil components can be used in food products which are used on a daily basis, and which have a possible contribution to health in general, and in particular to the lowering of blood cholesterol level.

In European patent application no. EP 619 952 (Amano), a food additive is described, by which the level of cholesterol in food is lowered by treating the food with the food additive. Upon using the additive, (y-oryzanol being mentioned), a complex with the cholesterol in the food is formed. The food containing the additive can therefor not be applied to lower the blood cholesterol level in mamals.

In International Application no. WO 92/19640 (Raision Marganiini oy) a substance of β-sitostanol fatty acid ester is described that can be used as such or added to food. It is described that an ordenary diet contains plant sterols 100-300 mg/day, and that these sterols are poorly absorbed from the intestines. In WO 92/19640, it is thus argued that the use of the plant sterols cannot be used for reducing the serum cholesterol levels in the human diet.

It has now been found that a lowering of blood cholesterol level is obtainable by the regular consumption of fat based food products as claimed in claim 1 which comprise phytosterol in an amount of at least 4 wt %, the weight percentages being based on the total weight of the fat based food product.

Thus, in another and preferred embodiment of the invention, it has now been found that a significant lowering of blood cholesterol level is obtainable by the regular consumption of fat based food products which comprise phytosterol.

Tocotrienol, phytosterol, and oryzanol are hereinafter also referred to as 'healthy components'.

The fat based food products of the present invention comprise preferably more than one compound of the group consisting of
tocotrienol, phytosterol, and oryzanol.

The amounts are amounts normally being sufficient to obtain statistically significant effects after use of these products in amounts similar to the amounts of the prior art fat based products, and for a prolonged period of time.

These amounts are based on common daily intake of the fat based food products. If more or less of such products are being used according to the particular use in a particular area or habits, the amounts can be adapted accordingly.

Tocotrienol, phytosterol, and oryzanol are compounds which are known per se. Japanese patent laid open number 04/320,645 describes the use of unsaponified substances of rice bran oil in rice bran oil, and mentions that the oil exhibited a very good physiological action. No substantiation of this is given.

In the present application, by the use of these components not only a cholesterol lowering effect, but also some antioxidative working can be found.

In a specific embodiment of the present invention, also at least one of tocopherol and polyphenol is present. These components are known as such, and have been described in different publications. For example, in "Tocopherole - Antioxidantien der Natur; by Pongracz et al.;Fat Sci. Technol.; 97. Jahrgang Nr. 3, pp. 90-104" a study of the use of tocopherols as antixoxidants in, inter alia, food is described.

Applicants have now found that the use of phytosterol results in a blood cholesterol lowering stronger than obtainable by the use of any pufa rich triglyceride fat blend alone.

As a further improvement of fat based food products comprising phytosterol, it was found that the use of a mixture of these healthy ingredients shows an effect on
health, in particular blood cholesterol lowering, which is much stronger than the effect which can be expected on the basis of the studies published regarding the effect of cholesterol lowering per weight amount of intake of each of these components alone. Moreover, this synergy was not found for blood cholesterol lowering alone, but also on other health aspects.
In particular, combinations of oryzanol, phytosterols and pufa rich glycerides show a very beneficial effect which could not be expected on the basis of the health effect of these compounds alone.

The oil related components used in the fat based foods of the present invention can be found as minor components in most plants from which oil is recovered, As conventional oil milling aims at producing triacylglycerols with a minimum amount of unsaponifiable matter, only limited amounts or none of the non-triglyceride healthy components at all are found in the oil presently available. The components to be used in the products of the present invention are found in the waste product streams which results upon the refining processes for obtaining commonly, classically refined oils.
As only very minor amounts of the suitable components are found in the waste streams of the usual oil refinings, e.g. sunflower oil, soybean oil, rapeseed oil, maizgerm oil and the like, using the waste of commonly used seed material will require enormous amounts of the waste material to be further process. However, relative significant amounts of one or more of these components can be found in materials not always used (yet) on a large scale as an fat or oil source. In particular, the components very suitable for the present invention can be found in, for example, rice bran (in particular oryzanol, phytosterols, and also tocopherols), wheat and maize germ (in particular phytosterols and tocotrienols), oat and oat bran in particular phytosterols and tocotrienols), sesame seed (in particular phytosterols), soybean , sheanuts (phytosterol) and palm oil (tocotrienols). The healthy minor oil components can also found, although in amounts lower than the sources mentioned before, in, for example, sunflower, rape, olive, line, peanuts, cotton, and safflower.

Oryzanol consist of a mixture of ferulic acid esters of unsaturated triterpene alcohols and is also referred to as gamma-oryzanol. In this invention only the term oryzanol is used. Within the group of tocopherols and tocotrienols one can distinguish alpha, beta, gamma and delta tocopherols and tocotrienols depending on the number and position of the methyl substituents on the chromane ring of the molecule. In this invention the terms tocopherol and tocotrienol is used to cover this whole family of molecules. For a further description and schematic drawing of the oryzanol and tocopherols and tocotrienols meant in this description, reference is made to "Separation of Vitamin E and gamma-Oryzanols from Rice Bran by Normal-Phase Chromatography", M. Diack and M. Saska, JAOCS Vol. 71, no. 11, pp. 1211. Phytosterols, also known as plant sterols or vegetable sterols can be classified in three groups, 4-desmethylsterols, 4-monomethylsterols and 4,4'-dimethylsterols. In oils they mainly exists as free sterols and sterol esters of fatty acids although sterol glucosides and acylated sterol glucosides are also present.

There are three major phytosterols namely beta-sitosterol, stigmasterol and campesterol Schematic drawings of the components meant are as given in "Influence of Processing on Sterols of Edible Vegetable Oils", S.P. Kochhar; Prog. Lipid Res. 22: pp. 161-188. In this invention the term phytosterol is used to cover the whole group of free phytosterols, phytosterol fatty acid esters and (acylated) phytosterol glucosides. The term polyphenols (or phenolics) can be defined chemically as a substance which possesses an aromatic ring bearing one or more hydroxy substituents, including functional derivatives. Reference is made to "Phenolic Compounds in Food", Chi-Tang Ho; Phenolic Compounds in Food and Their Effects on Health II, Am. Chemical Soc., 1992 In this invention the term polyphenols refers to all plant phenolic molecules derived from a plant source with an antioxidant activity and not covered by the terms oryzanol, tocotrienol and tocopherol, e.g. simple phenols and phenolic acids, hydroxycinnamic acid derivatives (e.g coumaric and ferulic acid) and flavonoids.

Tocopherol and polyphenol can also be obtained from most of these natural vegetable, oil related sources. These components can additionally be present where reference is made to 'healthy components'.

The healthy components can be added as single, pure components, as a mixture of such single, pure components, as components present in a concentrate obtained from specific processing of the natural source, as components obtained by mixing such concentrates (e.g., obtained from different sources) and the like.
The mixtures of the healthy oil components can be prepared by mixing single, pure components (bought at specialized suppliers) or be obtained by treating the oils and oil containing raw materials in such a way that the minor components remain therein. By specific processing, concentrates comprising a high amount of healthy minor oil components can be obtained. These are applied by preference if the 'naturalness' of a product is considered a particular issue. The concentration of the healthy minor oil components should be such that, if the concentrate is applied, the amounts indicated before are present in the fat based food product. Consequently, this means that the concentration should be high if it is desired to make a product in which the fat is replaced by non-fat ingredients, and a large amount of the other ingredients in the concentrate are fatty components.

Fat based food products are food products (partially) based on fat and regarded by the user, in particular the consumer, as 'fatty type of products'. Examples are yellow fat spreads (containing vegetable fat and/or animal fat such as butterfat), dressings, coffeecreamer, shortenings, cooking and frying oils, fillings and toppings, and the like. These products in most cases comprise a particular amount of fat. In some cases, however, products are still regarded as 'fatty type of products', despite a replacement of part or even all the fat by fat replacers. Fat based food products in which the fat is partially or completely replaced by fat replacers are also covered by the term fat based food products of this invention.
Fat products consisting of fat only are also considered as fat based food products in this specification. These are not only used as cooking and frying oils, but also sometimes in the industry in the preparation of food products, e.g. in baking.

The food products as such are common products in the western world, and are used by consumers on a daily basis in amounts different for each individual.
The invention is in particular very suitable for yellow fat spreads, dressings, cheese, shortenings and cooking and frying oils, and more in particular for yellow fat spreads which can comprise 0 (zero) to 90% fat (usually 5-80%). Dressings can comprise 0 to 85% faty (usually 5-80%), shortenings, cooking and frying oil more than 95% fat.

In a highly preferred embodiment, the food product is a yellow fat spread comprising 0 to 60% fat, preferably 0 to 40% fat, even more preferred 0 to 25% fat, the product comprising at least one of phytosterol and oryzanol and mixtures thereof, in an amount of at least 0.5 wt%, preferably at least 1wt% and more preferred at least 2 wt% for phytosterol, and at least 0.5 wt%, and preferably at least 1 wt% for oryzanol or their relative amounts if mixtures of one or more of these components are used, the weight percentages being based on the total weight of the fat based food product.

Yellow fat spreads comprising less than 40 wt% fat and at least 3 wt% phytosterol and 1.2 wt% oryzanol can be regarded as very beneficial in that these not only have a very low fat content, but also show an antioxidative effect and a significant lowering of the blood cholesterol working if applied similar to a common butterlike spread on a regular, daily basis. This applies even more for products with less than 25 wt% fat.

The use of the minor healthy components in cheese shows an additional beneficial effect, which is that the cholesterol in cheese is adsorbed by the body in a far lesser amount if phytosterol is present in the cheese than without. Hence, whereas cheese consumption usually adds cholesterol to the body, with the use of healthy minor oil components, in particular phytosterol, a strongly reduced absorbtion and a blood cholesterol lowering effect will be found. This effect can also be found for food products other than cheese comprising cholesterol or consumption of cholesterol containing food products in combination with the products as described in the invention. The amount of minor healthy components to be added on the preparation of the cheese is higher than the amount of cholesterol on a molar weight basis, so that the cheese product obtained still contains these minor healthy components in a free and active form.

The fat that is applied in these fat based food products can be any fat, such as dairy fat and/or vegetable fat. However, if fat is present, for health reasons the use of one or more vegetable fat sources is preferred. In particular, the use of liquid fats is preferred. These can be hydrogenated, interesterified, and the like. The fat can be one single fat or a blend. The fat or one of the fats applied can also beneficially be obtained from the same source as the healthy components are obtained from. E.g., rice bran oil can be obtained and mixed with one or more of the healthy components mentioned in this description which also are obtained from rice bran, or a mixture of wheat germ oil and rice bran oil can be used with one or more health components obtained from wheat germ and/or rice bran.

The use of fat compositions comprising a considerable amount of pufa rich triglycerides in addition to the use of the healthy components is in particular considered highly beneficial. For example, oils of sunflower, safflower, rapeseed, linseed, linola and/or soybean can be used in a preferred embodiment. Also the fat compositions mentioned in Netherlands patent documents no. Nl 143115, NL 178559, NL 155436, NL: 149687, NL 155177, European patent documents EP 41303, EP 209176, EP 249282, and EP 470658 are highly suitable.
If a fat blend is used, it is preferred that it comprises at least 30%, and more preferred at least 45% of poly- unsaturated fatty acids, based on the total weight amount of the fat in the fat based food product. So, a strong effect on the cholesterol lowering effect is obtained if use is made of a mixture of healthy minor oil components such as tocotrienol, phytosterol, and oryzanol, or extracts comprising these components, or these components in combination with the antioxidants polyphenols and tocopherols, or extracts comprising all of these these components in a food product in which a fat blend comprising at least 30 wt.% of pufa rich triglycerides is used.

E.g., sunflower oil can be obtained and mixed with one or more of the healthy components mentioned in this description which are obtained from rice bran, wheat germ or a mixture of wheat germ oil and rice bran oil. In another embodiment of the invention, it has been found that using specific technologies to incorporate the components in the food product significantly increased their physiological beneficial activity. In addition thereto, the solubility of the healty components in the food product will increase, and, as a result thereof, the maximum concentration can be increased without altering the product characteristics and quality.

The technigues which can be applied beneficially are incorporation into the fat by the use of micro-emulsions, liposomes, incorporation into micro-particles or adhering to micro-particles. According to another method, which is less preferred, one or more of the healthy components that might show a low solubility is esterified as desired with one or more fatty acids to increase their solubility. In the intestine these esters will be hydrolysed resulting in the free fatty acids and active minor oil component. Products so prepered are still considered, for the purpose of this invention, to contain the active minor oil component, as these are hydrolysed in the intestine.

The invention further envisages the use of an oil concentrate comprising more than 4 wt% of phytosterol for the preparation of a fat based food product as described above. In another preferred embodiment, the invention concerns the use of an oil concentrate comprising more than 4 wt% of phytosteral for the preparation of a yellow fat spread as described above.

### Example I

Male Syrian hamsters, aged 10-11 weeks, were fed semi-purified diets ad libitum, containing 30% of energy (en%) as fat; 23 en% as proteins; 47 en% as carbohydrates; 0.01% (w/w) cholesterol; and either 0.4% phytosterol preparation (ex Kaukas Oy Finland) alone or in combination with 100 ppm tocotrienols or nothing (control). The ratio of polyunsaturated (PUFA) to monounsaturated (MUFA) to saturated (SAFA) fatty acids in all the diets was either 7.2:12:10.8 (high PUFA) or 3:12:15 (high SAFA). The semipurified diets were fed for 6 weeks, after a run-in period on the control diet for 2 weeks. The experimental groups comprised 12-14 hamsters/group. The hamsters were housed individually in Makrolon cages type II, with a layer of sawdust as bedding. At the end of the experiment blood samples were collected and (heparin) plasma total cholesterol was determined using a commercially available enzymic method (CHOD-PAP).

Throughout the experiment body weights and feed intake did not differ between the experimental groups. No clinical signs were observed in any group. The average total plasma cholesterol in the high SAFA control groups was 4.25 mM. A decrease on average total plasma cholesterol in the high PUFA control group of 0.30 mM was observed. Addition of phytosterols decreased the average total plasma cholesterol with 0.94 and 0.95 mM for the high SAFA and high PUFA diets respectively. With additional tocotrienols, no additional decreased on the average total plasma cholesterol could be found.

## Claims

1. Fat based food product wherein the food product is a yellow fat spreads containing vegetable fat and/or animal fat such as butterfat, a dressing, a coffeecreamer, a shortening, a filling or a topping, comprising natural fat components which have a blood cholesterol lowering effect in amounts sufficient to obtain a blood cholesterol lowering effect, wherein the product comprises at least 4 wt% phytosterol weight percentages being based on the total weight of the fat based food product, wherein the fat used in the product is a fat comprising at least 30 wt%, and preferably at least 45wt% of pufa rich triglycerides, calculated on the total weight of the fat present in the product.

2. Fat based food product according to claim 1, wherein also at least one compound of the group consisting of tocopherol and polyphenol is present.

3. Fat based food product according to any one of claims 1 to 2, wherein the fat based food product is a yellow fat spread.

## Patentansprüche

1. Lebensmittelprodukt auf Fettbasis, wobei das Lebensmittelprodukt ein gelber Fettaufstrich, der Pflanzenfett und/oder Tierfett, z.B. Butterfett, enthält, ein Dressing, ein Kaffeeweißer, ein Shortening, eine Füllung oder eine Beschichtung ist, das natürliche Fettkomponenten, die eine Blutcholesterin-senkende Wirkung haben, in zur Erreichung einer Blutcholesterin-senkenden Wirkung ausreichenden Mengen umfasst, wobei das Produkt wenigstens 4 Gew.-% Phytosterol umfasst und die Gewichtsprozentangaben auf das Gesamtgewicht des Lebensmittelproduktes auf Fettbasis bezogen sind, wobei das in dem Produkt verwendete Fett ein Fett ist, das wenigstens 30 Gew.-% und vorzugsweise wenigstens 45 Gew.-% PUFA-reiche Triglyceride, berechnet auf das Gesamtgewicht des im Produkt vorliegenden Fetts, umfasst.

2. Lebensmittelprodukt auf Fettbasis nach Anspruch 1, in dem wenigstens eine Verbindung der Gruppe, die aus Tocopherol und Polyphenol besteht, vorhanden ist.

3. Lebensmittelprodukt auf Fettbasis nach einem der Ansprüche 1 bis 2, wobei das Lebensmittelprodukt auf Fettbasis ein gelber Fettaufstrich ist.

## Revendications

1. Produit alimentaire à base de graisse dans lequel le produit alimentaire est une pâte à tartiner jaune contenant de la graisse animale et/ou végétale telle que de la matière grasse du beurre, une sauce vinaigrette, un blanchissant pour le café, une graisse pour la pâtisserie, une garniture ou un enrobage, comprenant des composants de graisse naturelle ayant un effet de réduction du cholestérol sanguin, dans des quantités suffisantes pour obtenir un effet de réduction du cholestérol dans le sang, dans lequel le produit contient au moins 4 % en poids de phytostérol, les pourcentages en poids se basant sur le poids total de graisse dans le produit, dans lequel la graisse utilisée dans le produit est une graisse comprenant au moins 30 % en poids, de préférence au moins 45 % en poids de triglycérides riches en acides gras poly-insaturés, calculé sur le poids total de la graisse présente dans le produit.

2. Produit alimentaire à base de graisse selon la revendication 1, dans lequel est également présent au moins un composé du groupe constitué du tocophérol et du polyphénol.

3. Produit alimentaire à base de graisse selon l'une quelconque des revendications 1 à 2, dans lequel le produit alimentaire à base de graisse est une pâte à tartiner jaune.
